# EUROPÄISCHE PATENTSCHRIFT

(11) **EP 1 991 529 B1**
(45) Veröffentlichungstag und Bekanntmachung des Hinweises auf die Patenterteilung: **09.12.2009**
(21) Anmeldenummer: 07722932.6
(22) Anmeldetag: 26.02.2007
(51) Int. Cl.: C07D 213/73, C07D 413/04, A61K 31/4439, A61K 31/44, A61P 31/00

(54) **SUBSTITUIERTE ARYLSULFONAMIDE ALS ANTIVIRALE MITTEL**
SUBSTITUTED ARYLSULFONAMIDES AS ANTIVIRAL AGENTS
ARYLSULFONAMIDES SUBSTITUÉS UTILISÉS EN TANT QU'AGENTS ANTIVIRAUX

(30) Priorität: 03.03.2006 DE 102006009928
(43) Veröffentlichungstag der Anmeldung: 19.11.2008
(73) Patentinhaber: AiCuris GmbH & Co. KG, 42117 Wuppertal (DE)
(72) Erfinder: SVENSTRUP, Niels, 42553 Velbert (DE); ZIMMERMANN, Holger, 42111 Wuppertal (DE); KARTHAUS, Dagmar, 42697 Solingen (DE); GOELLER, Andreas, 42113 Wuppertal (DE); HEIMBACH, Dirk, 40629 Düsseldorf (DE); HENNINGER, Kerstin, 42115 Wuppertal (DE); LANG, Dieter, 42553 Velbert (DE); PAULSEN, Daniela, 42105 Wuppertal (DE); RIEDL, Bernd, 42329 Wuppertal (DE); SCHOHE-LOOP, Rudolf, 42327 Wuppertal (DE); SCHUHMACHER, Joachim, 42113 Wuppertal (DE); WUNBERG, Tobias, A-2371 Hinterbruehl (AT)
(74) Vertreter: Scheuermann, Erik
(86) Internationale Anmeldenummer: PCT/EP2007/001620
(87) Internationale Veröffentlichungsnummer: WO 2007/101573

(56) Entgegenhaltungen:
- WO-A-02/085869

## Beschreibung

Die Erfindung betrifft substituierte Arylsulfonamide und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

WO 02/085869 beschreibt substituierte Arylsulfonamide als antivirale Mittel, insbesondere gegen Cytomegaloviren.

Eine Aufgabe der vorliegenden Erfindung ist es, neue Verbindungen mit gleicher oder verbesserter antiviraler Wirkung, verbesserter Pharmakokinetik, insbesondere längerer Halbwertszeit und/oder verbesserter oraler Bioverfügbarkeit, und deren metabolische Abbauwege sich zwischen Mensch und gängigen Tox-Spezies wie z.B. Ratte und Hund nicht signifikant unterscheiden zur Behandlung von viralen Infektionskrankheiten bei Menschen und Tieren zur Verfügung zu stellen.

Überraschenderweise wurde gefunden, dass die in der vorliegenden Erfindung beschriebenen substituierten Arylsulfonamide antiviral wirksam sind, verbesserte pharmakokinetische Eigenschaften zeigen und deren metabolische Abbauwege sich in Mensch, Ratte und Hund nicht signifikant unterscheiden.

Gegenstand der Erfindung sind Verbindungen der Formel 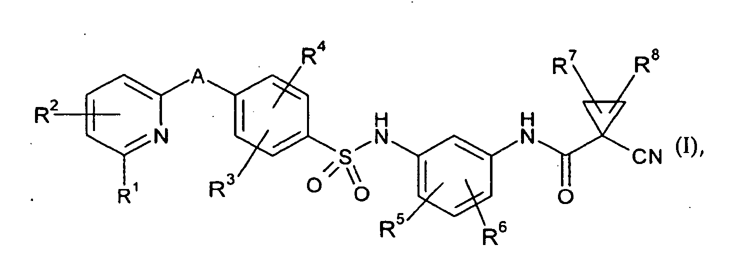 in welcher
- A: für eine Gruppe der Formel 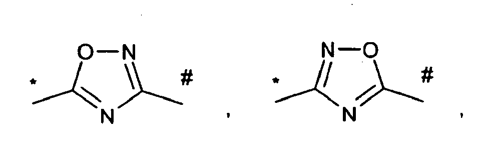 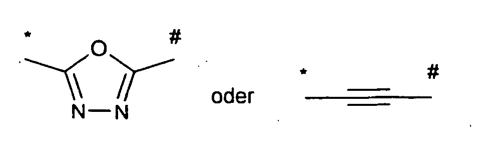 steht,
wobei
*die Anknüpfstelle an das Kohlenstoffatom des Pyridinyl-Rings ist,
und
# die Anknüpfstelle an das Kohlenstoffatom des Phenyl-Rings ist,
- R¹: für Wasserstoff, Amino oder Methylcarbonylamino steht,
- R²: für Wasserstoff oder Halogen steht,
- R³: für Wasserstoff, Halogen oder Cyano steht,
- R⁴: für Wasserstoff, Halogen oder Cyano steht,
- R⁵: für Wasserstoff oder Halogen steht,
- R⁶: für Wasserstoff oder Halogen steht,
- R⁷: für Wasserstoff, Halogen oder C₁-C₃-Alkyl steht,
- R⁸: für Wasserstoff, Halogen oder C₁-C₃-Alkyl steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht für einen linearen oder verzweigten Alkylrest mit in der Regel 1 bis 3, besonders bevorzugt 1 bis 2 Kohlenstoffatomen, beispielhaft und vorzugsweise für Methyl, Ethyl, n-Propyl und Isopropyl.
Halogen steht für Fluor, Chlor, Brom und Jod, bevorzugt Fluor und Chlor.

In der Formel der Gruppe, die für A stehen kann, steht der Endpunkt der Linie, neben der jeweils ein *oder # steht, nicht für ein Kohlenstoffatom beziehungsweise eine CH₂-Gruppe, sondern ist Bestandteil der Bindung zu dem Atom, an das A gebunden ist.

Bevorzugt sind Verbindungen der Formel (I), in welcher
- A: für eine Gruppe der Formel 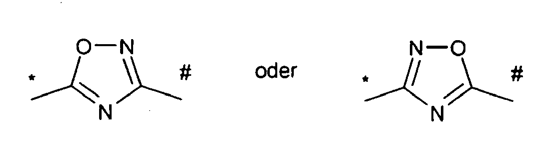 steht,
wobei
* die Anknüpfstelle an das Kohlenstoffatom des Pyridinyl-Rings ist,
und
# die Anknüpfstelle an das Kohlenstoffatom des Phenyl-Rings ist,
- R¹: für Wasserstoff, Amino oder Methylcarbonylamino steht,
- R², R³ und R⁴: für Wasserstoff stehen,
- R⁵: für Wasserstoff oder Halogen steht,
- R⁶: für Wasserstoff oder Halogen steht,
- R⁷ und R⁸: für Wasserstoff stehen,
und ihre Salze, ihre Solvate und die Solvate ihrer Salze.

Bevorzugt sind auch Verbindungen der Formel (I), in welcher
- A: für eine Gruppe der Formel 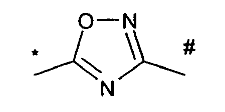 steht,
wobei
* die Anknüpfstelle an das Kohlenstoffatom des Pyridinyl-Rings ist,
und
# die Anknüpfstelle an das Kohlenstoffatom des Phenyl-Rings ist,
- R¹: für Amino oder Methylcarbonylamino steht,
- R², R³ und R⁴: für Wasserstoff stehen,
- R⁵: für Wasserstoff steht,
- R⁶: für Wasserstoff oder Halogen steht,
- R⁷ und R⁸: für Wasserstoff stehen,
und ihre Salze, ihre Solvate und die Solvate ihrer Salze.

Bevorzugt sind auch Verbindungen der Formel (I), in welcher
- A: für eine Gruppe der Formel 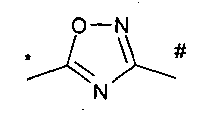 steht,
wobei
* die Anknüpfstelle an das Kohlenstoffatom des Pyridinyl-Rings ist,
und
# die Anknüpfstelle an das Kohlenstoffatom des Phenyl-Rings ist.

Bevorzugt sind auch Verbindungen der Formel (I), in welcher R¹ für Amino steht.

Bevorzugt sind auch Verbindungen der Formel (I), in welcher R² für Wasserstoff steht.

Bevorzugt sind auch Verbindungen der Formel (I), in welcher R³ für Wasserstoff steht.

Bevorzugt sind auch Verbindungen der Formel (I), in welcher R⁴ für Wasserstoff steht.

Bevorzugt sind auch Verbindungen der Formel (I), in welcher R⁵ für Wasserstoff steht.

Bevorzugt sind auch Verbindungen der Formel (I), in welcher R⁶ für Fluor steht.

Bevorzugt sind auch Verbindungen der Formel (I), in welcher R⁵ für Wasserstoff und R⁶ für Fluor steht.

Bevorzugt sind auch Verbindungen der Formel (I), in welcher R⁷ für Wasserstoff steht.

Bevorzugt sind auch Verbindungen der Formel (I), in welcher R⁸ für Wasserstoff steht.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei Verbindungen der Formel 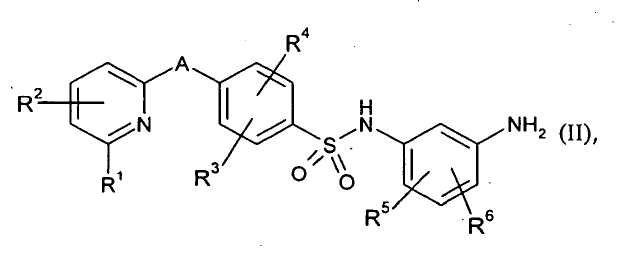 in welcher
- A, R¹, R², R³, R⁴, R⁵ und R⁶: die oben angegebene Bedeutung haben,
mit Verbindungen der Formel 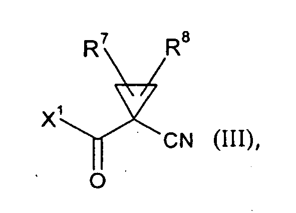 in welcher
- R⁷ und R⁸: die oben angegebene Bedeutung haben, und
- X¹: für Halogen, bevorzugt Chlor oder Brom, oder Hydroxy steht,
umgesetzt werden.

Die Umsetzung erfolgt, falls X¹ gleich Halogen ist, im Allgemeinen in inerten Lösungsmitteln, in Gegenwart einer Base, bevorzugt in einem Temperaturbereich von 0°C bis 40°C bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösungsmittel wie Aceton, Dimethylformamid, Dimethylacetamid, 2-Butanon oder Acetonitril, bevorzugt ist Tetrahydrofuran oder Methylenchlorid.

Basen sind beispielsweise Alkalicarbonate wie Cäsiumcarbonat, Natrium- oder Kaliumcarbonat, oder organische Basen wie Trialkylamine, z.B. Triethylamin oder Diisopropylethylamin, oder *N*-Methylmorpholin, *N*-Methylpiperidin, 4-Dimethylaminopyridin oder Pyridin, bevorzugt ist Diisopropylethylamin.

Die Umsetzung erfolgt, falls X¹ gleich Hydroxy ist, im Allgemeinen in inerten Lösungsmitteln, in Gegenwart von Dehydratisierungsreagenzien, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylaminoisopropyl)-*N'*-ethylcarbodiimid-Hydrochlorid (EDC) (gegebenenfalls in Gegenwart von Pentafluorphenol (PFP)), *N*-Cyclohexylcarbodiimid-*N'-*propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyluroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen, mit Basen. Vorzugsweise wird die Kondensation mit HATU durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin oder Diisopropylethylamin, oder *N*-Methylmorpholin, *N*-Methylpiperidin oder 4-Dimethylaminopyridin, bevorzugt ist Diisopropylethylamin.

Inerte Lösungsmittel sind beispielsweise Halogenkohlenwasserstoffe wie Dichlormethan oder Trichlormethan, Kohlenwasserstoffe, wie z.B. Benzol, oder Nitromethan, Dioxan, Dimethylsulfoxid, Dimethylformamid, Acetonitril, Tetrahydrofuran oder Hexamethylphosphorsäuretriamid, oder Gemische der Lösungsmittel, besonders bevorzugt ist Dichlormethan, Tetrahydrofuran oder Dimethylformamid.

Die Verbindungen der Formel (III) sind bekannt oder lassen sich nach bekannten Verfahren aus den entsprechenden Edukten synthetisieren.

Die Verbindungen der Formel (II) sind bekannt oder können hergestellt werden, indem Verbindungen der Formel 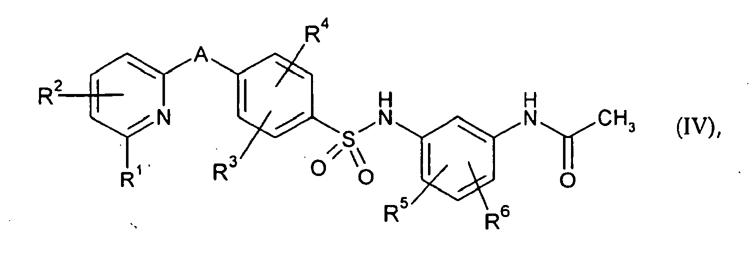 in welcher
A, R¹, R², R³, R⁴, R⁵ und R⁶ die oben angegebene Bedeutung haben,
mit einer Säure umgesetzt werden.

Die Umsetzung erfolgt im Allgemeinen in polaren Lösungsmitteln, bevorzugt in einem Temperaturbereich von Raumtemperatur bis zum Rückfluss der Lösungsmittel bei Normaldruck.

Säuren sind beispielsweise Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure oder Trifluoressigsäure, besonders bevorzugt ist Chlorwasserstoffsäure.

Polare Lösungsmittel sind beispielsweise Alkohole wie Methanol, Ethanol, n-Propanol, iso-Propanol, n-Butanol oder *tert-*Butanol, oder Tetrahydrofuran, Dioxan oder Essigsäure, oder Gemische der Lösungsmittel oder ein Gemisch aus Lösungsmittel und Wasser, besonders bevorzugt ist Ethanol.

Die Verbindungen der Formel (IV) sind bekannt oder können hergestellt werden, indem nach Verfahren
[A] Verbindungen der Formel 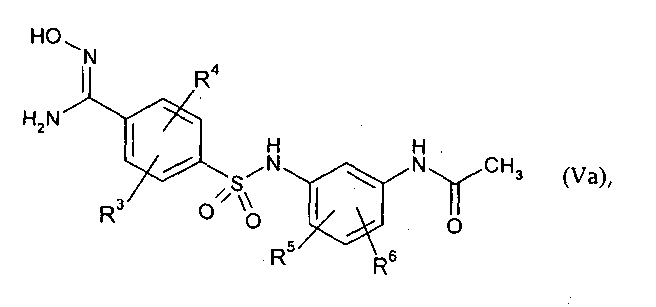 in welcher
   R³, R⁴, R⁵ und R⁶ die oben angegebene Bedeutung haben,
   mit Verbindungen der Formel 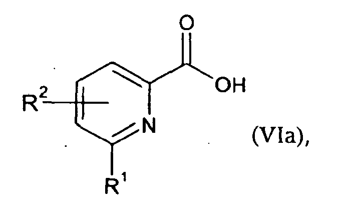 in welcher
   R¹ und R² die oben angegebene Bedeutung haben,
   zu Verbindungen der Formel 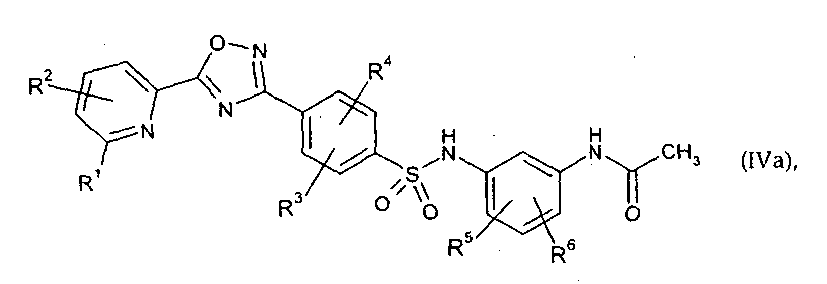 in welcher
   R¹, R², R³, R⁴, R⁵ und R⁶ die oben angegebene Bedeutung haben,
   umgesetzt werden
   oder
[B] Verbindungen der Formel 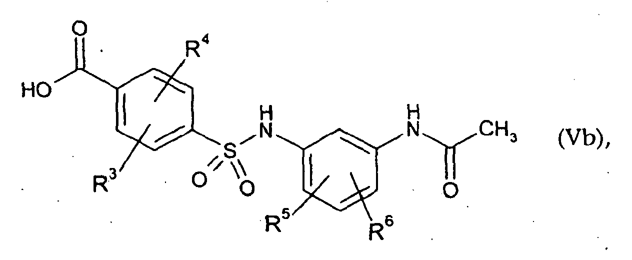 in welcher
   R³, R⁴, R⁵ und R⁶ die oben angegebene Bedeutung haben,
   mit Verbindungen der Formel 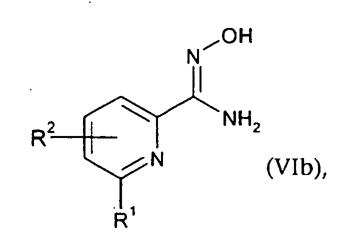 in welcher
   R¹ und R² die oben angegebene Bedeutung haben,
   zu Verbindungen der Formel 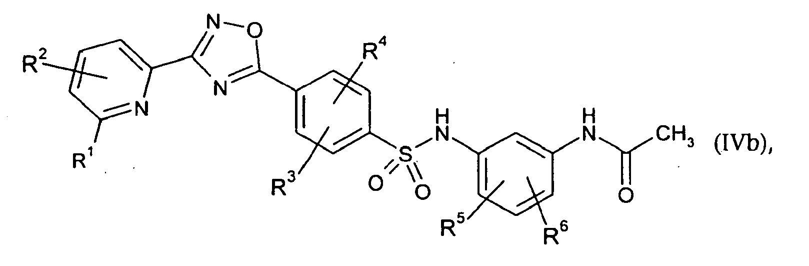 in welcher
   R¹, R², R³, R⁴, R⁵ und R⁶ die oben angegebene Bedeutung haben,
   umgesetzt werden
   oder
[C] Verbindungen der Formel (Vb) in der ersten Stufe mit Verbindungen der Formel 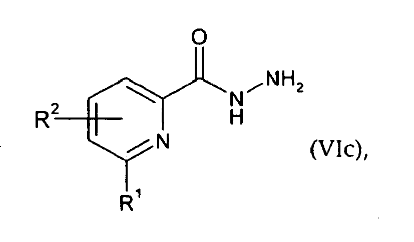 in welcher
   R¹ und R² die oben angegebene Bedeutung haben,
   und in der zweiten Stufe mit Phosphoroxychlorid, zu Verbindungen der Formel 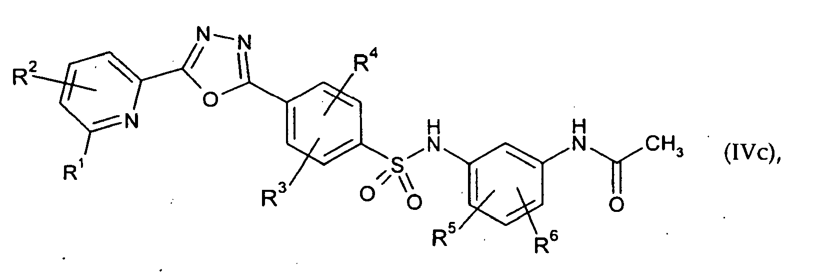 in welcher
   R¹, R², R³, R⁴, R⁵ und R⁶ die oben angegebene Bedeutung haben,
   umgesetzt werden
   oder
[D] Verbindungen der Formel 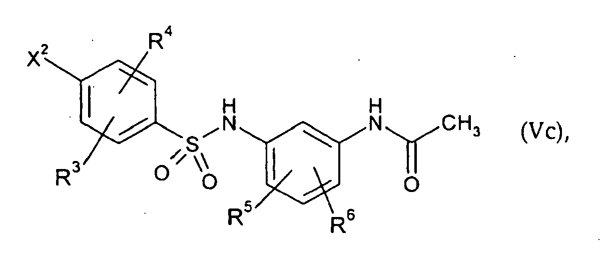 in welcher
   R³, R⁴, R⁵ und R⁶ die oben angegebene Bedeutung haben, und
   X² für Halogen, bevorzugt Iod oder Brom steht,
   mit Verbindungen der Formel 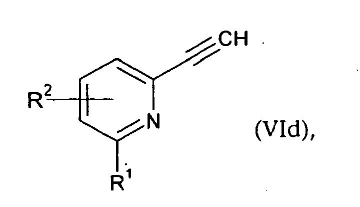 in welcher
   R¹ und R² die oben angegebene Bedeutung haben,
   zu Verbindungen der Formel 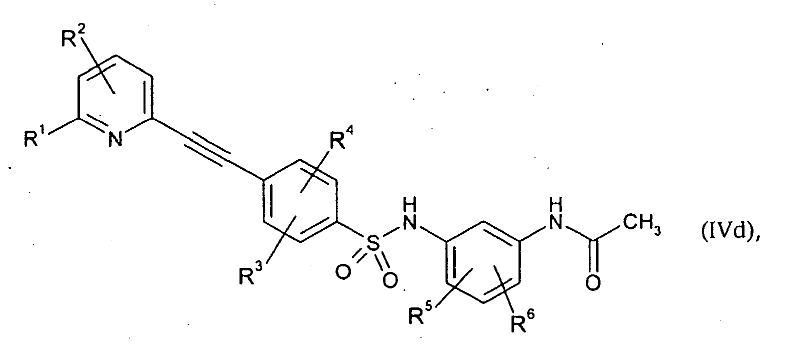 in welcher
   R¹, R², R³, R⁴, R⁵ und R⁶ die oben angegebene Bedeutung haben,
   umgesetzt werden.

Die Aminogruppe von R¹ ist gegebenenfalls während der Synthese mit einer dem Fachmann bekannten Schutzgruppe, wie z.B. Acyl geschützt, die nach der Synthese nach dem Fachmann bekannten Bedingungen abgespalten wird.

Die Verbindungen der Formeln (IVa), (IVb), (IVc) und (IVd) bilden zusammen die Verbindungen der Formel (IV).

Die Umsetzung nach Verfahren [A], [B] und der ersten Stufe nach Verfahren [C] erfolgt im Allgemeinen in inerten Lösungsmitteln in Gegenwart von Dehydratisierungsreagenzien, bevorzugt in einem Temperaturbereich von Raumtemperatur bis 100°C bei Normaldruck.

Inerte Lösungsmittel sind beispielsweise Kohlenwasserstoffe wie Benzol oder Toluol, oder andere Lösungsmittel wie Dioxan, Dimethylformamid, Dimethylsulfoxid oder Acetonitril, oder Gemische der Lösungsmittel, besonders bevorzugt ist Dimethylformamid.

Dehydratisierungsreagenzien sind beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylaminoisopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder Mischungen aus diesen, mit Basen. Besonders bevorzug ist Carbonyldiimidazol.

Die Umsetzung der zweiten Stufe nach Verfahren [C] erfolgt im Allgemeinen in inerten Lösungsmitteln, bevorzugt in einem Temperaturbereich von 50°C bis 100°C bei Normaldruck. Ebenso ist es möglich, Gemische der Lösungsmittel, ein Gemisch aus Lösungsmittel und POCl₃ oder reines POCl₃ einzusetzen.

Inerte Lösungsmittel sind beispielsweise Kohlenwasserstoffe wie Benzol oder Toluol, oder andere Lösungsmittel wie Dioxan, Dimethylsulfoxid, Dimethylformamid oder Acetonitril, oder Gemische der Lösungsmittel, besonders bevorzugt ist Dioxan und/oder Dimethylformamid.

Die Umsetzung nach Verfahren [D] erfolgt im Allgemeinen unter Sonogashira-Reaktionsbedingungen unter Argon in inerten und entgasten Lösungsmitteln, in Gegenwart eines Katalysators, gegebenenfalls in Gegenwart eines Zusatzreagenzes, in Anwesenheit einer Base und gegebenenfalls Triphenylphosphin, bevorzugt in einem Temperaturbereich von Raumtemperatur bis zum Rückfluss des Lösungsmittels bei Normaldruck (R.R. Tykwinski, Angew. Chem. Int. Ed. 2003, 42, 1566-1568, K. Sonogashira in Handbook of organopalladium chemistry for organic synthesis (Ed. E.-I. Negishi), 1133-1178 Wiley-Interscience, New-York (2002)).

Katalysatoren sind beispielsweise für Sonogashira-Reaktionsbedingungen übliche Palladium-Katalysatoren, bevorzugt sind Katalysatoren wie z.B. Tri(dibenzylidenaceton)dipalladium, Dichlorbis(triphenylphosphin)-palladium, Tetrakistriphenylphosphinpalladium(0), Palladium(II)-acetat, 1,1'-Bis[(diphenylphosphino)-ferrocen]palladium-II-chlorid (1:1)-Komplex mit Dichlormethan.

Zusatzreagenzien sind beispielsweise Kupfer-(I)-iodid und Triphenylphosphin.

Basen sind beispielsweise Aminbasen wie Triethylamin.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder andere Lösungsmittel wie Nitrobenzol, Dimethylformamid, Dimethylacetamid, Dimethylsulfoxid oder N-Methylpyrrolidon, bevorzugt sind Lösungsmittel wie z.B. Dimethylformamid, Dimethylacetamid, Dimethylsulfoxid oder 1,2-Dimethoxyethan.

Die Verbindungen der Formeln (VIa), (VIb), (VIc) und (VId) sind bekannt oder lassen sich nach bekannten Verfahren aus den entsprechenden Edukten synthetisieren.

Die Verbindungen der Formeln (Va), (Vb) und (Vc) sind bekannt oder können hergestellt werden, indem Verbindungen der Formel 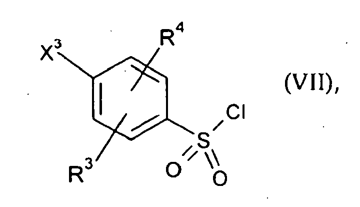 in welcher
- R³ und R⁴: die oben angegebene Bedeutung haben, und
- X³: für Halogen, bevorzugt Iod oder Brom, Hydroxycarbonyl oder Cyano steht,
mit Verbindungen der Formel 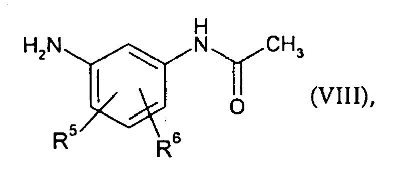 in welcher
- R⁵ und R⁶: die oben angegebene Bedeutung haben,
umgesetzt werden.

Die Umsetzung erfolgt im Allgemeinen in inerten Lösungsmitteln in Gegenwart einer Base, bevorzugt in einem Temperaturbereich von 0°C bis 40°C bei Normaldruck.

Inerte Lösungsmittel sind beispielsweise Alkohole wie Methanol, Ethanol, n-Propanol, iso-Propanol, n-Butanol oder *tert-*Butanol, oder Tetrahydrofuran, Aceton, Dioxan oder Pyridin, oder Gemische der Lösungsmittel oder ein Gemisch aus Lösungsmittel und Wasser, besonders bevorzugt ist Tetrahydrofuran oder iso-Propanol mit etwas Wasser.

Basen sind beispielsweise Natriumacetat, Kaliumacetat, Natriumcarbonat, Kaliumcarbonat oder Aminbasen wie Triethylamin oder Diisopropylethylamin, besonders bevorzugt ist Natriumacetat.

Die Verbindungen der Formeln (VII) und (VIII) sind bekannt oder lassen sich nach bekannten Verfahren aus den entsprechenden Edukten synthetisieren.

In einem alternativen Verfahren können die Verbindungen der Formel (IV) durch eine andere Reihenfolge der Kupplung der Synthesebausteine hergestellt werden.

Die Herstellung der erfindungsgemäßen Verbindungen kann durch die folgenden Syntheseschemata verdeutlicht werden.

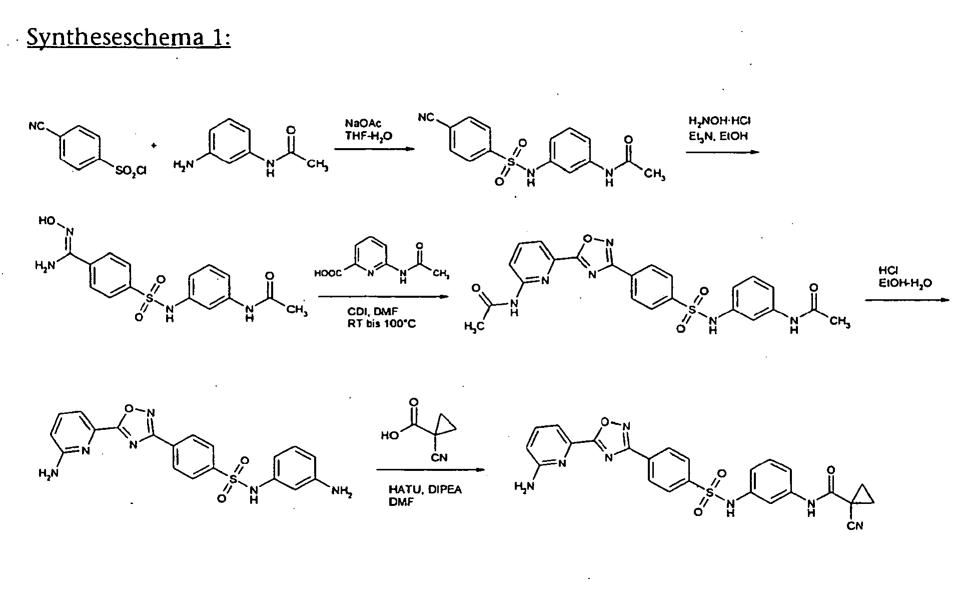

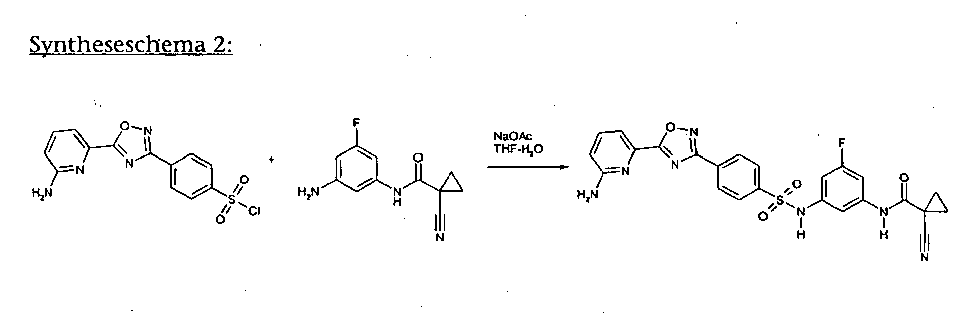

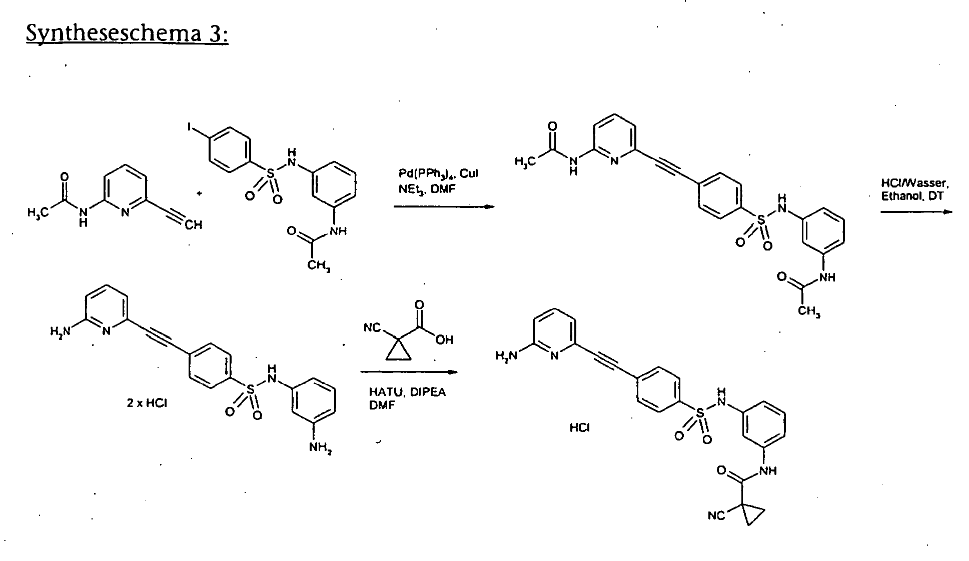

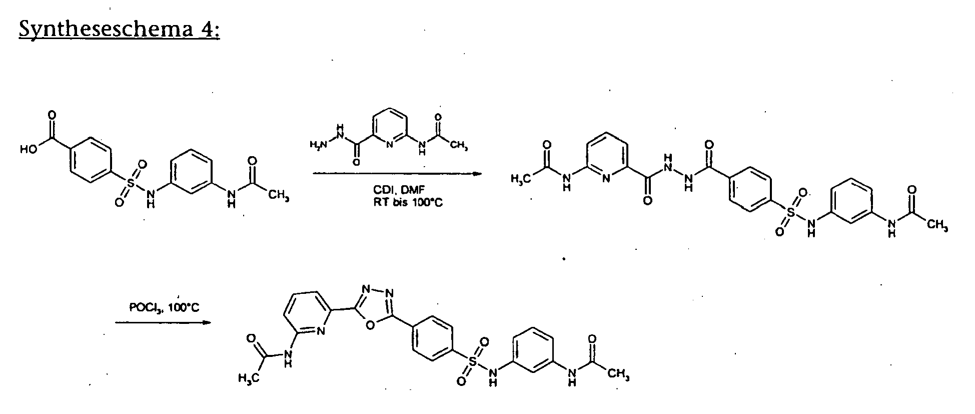

Die erfindungsgemäßen Verbindungen zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.
6) Behandlung von HCMV-positiven Krebspatienten mit dem Ziel, HCMV-vermittelte Tumorprogression zu verringern (vgl. J. Cinatl, et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung ist Verwendung einer antiviral wirksamen Menge der erfindungsgemäßen Verbindungen zur Herstellung eines Medikaments zur Behandlung und/oder Prophylaxe von Erkrankungen, insbesondere der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Valgancyclovir, Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch, topisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- ca.: circa
- Boc: tert-Butoxycarbonyl
- CDCl₃: Deuterochloroform
- DCI: direkte chemische Ionisation (bei MS)
- DCM: Dichlormethan
- DIEA: *N,N*-Diisopropylethylamin
- DMSO: Dimethylsulfoxid
- DMF: *N,N*-Dimethylformamid
- d. Th.: der Theorie
- EDC: *N*-(3-Dimethylaminoisopropyl)-*N'*-ethylcarbodiimid- hydrochlorid
- EE: Ethylacetat (Essigsäureethylester)
- EI: Elektronenstoß-Ionisation (bei MS)
- ESI: Elektrospray-Ionisation (bei MS)
- Fmoc: 9-Fluorenylmethoxycarbonyl
- ges.: gesättigt
- h: Stunde
- HPLC: Hochdruck-, Hochleistungsflüssigchromatographie
- HV: Hochvakuum
- konz.: konzentriert
- LC-MS: Flüssigchromatographie-gekoppelte Massenspektroskopie
- LDA: Lithium-Diisopropylamid
- min: Minuten
- MS: Massenspektroskopie
- MTBE: Methyl-*tert-*butylether
- NMR: Kernresonanzspektroskopie
- Pd-C: Palladium auf Kohle
- proz.: prozentig
- PyBOP: 1-Benzotriazolyloxy- tripyrrolidinophosphoniumhexafluorophosphat
- RP-HPLC: Reverse Phase HPLC
- RT: Raumtemperatur
- Rₜ: Retentionszeit (bei HPLC)
- THF: Tetrahydrofuran

### Allgemeine Methoden LC-MS und HPLC:

**Methode 1 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 2(LC-MS)**: Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 3 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (LC-MS):** Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 5 (HPLC):** Instrument: HP 1100 mit DAD-Detektion; Säule: Kromasil RP-18, 60 mm x 2 mm, 3.5 µm; Eluent A: 5 ml Perchlorsäure/l Wasser, Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Ofen: 30°C; UV-Detektion: 210 nm.

**Methode 6 (HPLC):** Instrument: HP 1100 mit DAD-Detektion; Säule: Kromasil RP-18, 60 mm x 2 mm, 3.5 µm; Eluent A: 5 ml Perchlorsäure/l Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Ofen: 30°C; UV-Detektion: 210 nm.

**Methode 7 (LC/MS):** Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

### Ausgangsverbindungen

### Beispiel 1A

### 4-(Benzylthio)benzonitril

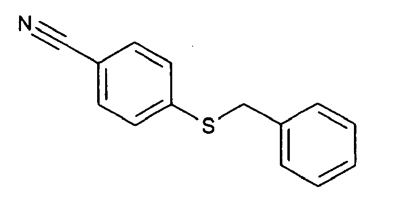

Natriumhydrid (5 g einer 60%-igen Dispersion in Öl) wird mit Hexan gewaschen und im Vakuum getrocknet. Der Rückstand wird in trockenem DMF (100 ml) bei 0°C aufgeschlemmt und Benzylmercaptan (14.82 g) über 30 min zugetropft. Anschließend wird 30 min bei Raumtemperatur nachgerührt. Man setzt vorsichtig 4-Fluorbenzonitril (14.45 g) zu und rührt das Reaktionsgemisch nach, bis das Ausgangsmaterial komplett umgesetzt ist (HPLC Kontrolle, ca. 3 Stunden). Das Reaktionsgemisch wird auf Eiswasser (400 ml) gegossen und fünf Minuten nachgerührt. Das Produkt wird abfiltriert, mit Wasser (dreimal) gewaschen und auf dem Filter getrocknet. Das Rohprodukt wird aus Cyclohexan umkristallisiert, abfiltriert und mit Petrolether gewaschen und getrocknet. Man erhält 23.04 g (86% d. Th.) Produkt.
LC-MS (Methode 1): Rₜ = 2.85 min
MS (ESI): m/z = 226 [M+H]⁺

### Beispiel 2A

### 4-(Benzylthio)-N'-hydroxybenzocarboximidamid

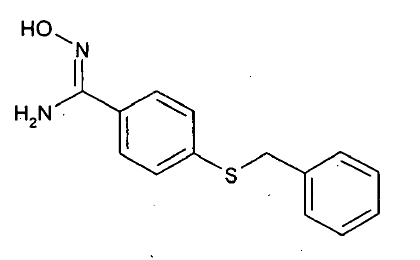

4-(Benzylthio)benzonitril (23.00 g) und Hydroxylamin Hydrochlorid (10.66 g) werden in trockenem Ethanol (10 ml) vorgelegt und Triethylamin (17 ml) wird zugesetzt. Das Reaktionsgemisch wird erst 30 min bei 50°C gerührt und danach 2 Stunden unter Rückfluss erhitzt. Anschließend wird Wasser zugesetzt bis die Lösung trüb wird. Das Reaktionsgemisch wird auf RT abgekühlt und der resultierende Feststoff abfiltriert. Der Feststoff wird mit Wasser gewaschen und anschließend bei 85°C im Trockenschrank getrocknet. Das Rohprodukt wird aus n-Butanol umkristallisiert, das kristalline Produkt abfiltriert, mit Diethylether gewaschen und im Trockenschrank bei 65°C getrocknet. Man erhält 23.40 g (88% d. Th.) des Produktes als Feststoff.
LC-MS (Methode 1): Rₜ = 1.79 min
MS (ESI): m/z = 229 [M+H]+

### Beispiel 3A

### N-(6-{3-[4-(Benzylthio)phenyl]-1,2,4-oxadiazol-5-yl}pyridin-2-yl)acetamid

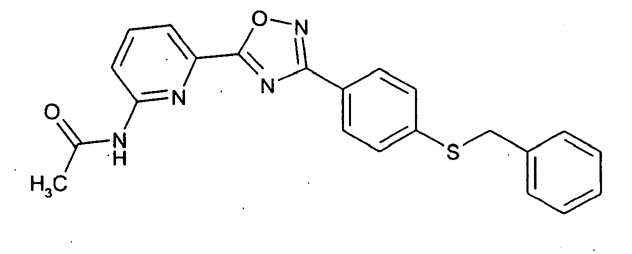

Zu 6-Acetamidopyridin-2-carbonsäure (16.84 g) in trockenem DMF (75 ml) wird 1,1-Carbonyldiimidazol (15.16 g) langsam in kleinen Portionen zugesetzt (Gasentwicklung). Die resultierende Lösung wird 1.5 Stunden bei RT gerührt. Dann wird 4-(Benzylthio)-N'-hydroxybenzo-carboximidamid (23.00 g) zugegeben und das Reaktionsgemisch bei RT gerührt bis sich das Ausgangsmaterial komplett umgesetzt hat (ca. 3 Stunden). Das Reaktionsgemisch wird auf 100°C erwärmt und 2 Stunden nachgerührt. Anschließend wird Wasser zugesetzt bis eine leichte Trübung einsetzt und das Reaktionsgemisch auf RT abgekühlt. Das Rohprodukt wird abfiltriert, dreimal mit Wasser gewaschen und bei 65°C im Trockenschrank getrocknet. Man erhält 24.42 g (67% d. Th.) des Produktes als Feststoff.
LC-MS (Methode 1): Rₜ = 2.98 min
MS (ESI): m/z = 403 [M+H]⁺
¹H-NMR (400 MHz, DMSO-d₆): δ = 10.96 (s, 1H), 8.38 (d, 1H), 8.08 (t, 1H), 8.02-7.95 (m,3H), 7.53 (d, 2H), 7.43 (d, 2H), 7.35-7.21 (m, 3H), 4.36 (s, 2H), 2.15 (s, 3H).

### Beispiel 4A

### 6-(3-[4-(Benzylthio)phenyl]-1,2,4-oxadiazol-5-yl}pyridin-2-amin Hydrochlorid

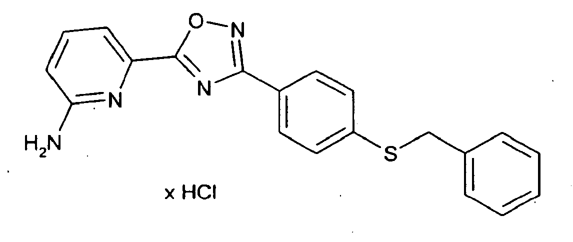

Zu N-(6-{3-[4-(Berizylthio)phenyl]-1,2,4-oxadiazol-5-yl}pyridin-2-yl)acetamid (40.55 g) in Ethanol (150 ml) werden Wasser (50 ml) und konzentrierte Salzsäure (50 ml) zugesetzt. Das Reaktionsgemisch wird unter Rückfluss erhitzt bis das Ausgangsmaterial komplett umgesetzt ist (ca. 3 Stunden) und anschließend wird auf RT abgekühlt. Der Feststoff wird abfiltriert, dreimal mit Ethanol gewaschen und im Vakuumofen bei 80°C getrocknet. Man erhält 36.60 g (92% d. Th.) des Produktes als Feststoff.
LC-MS (Methode 2): Rₜ = 2.76 min
MS (ESI): m/z = 361 [M+H]⁺
¹H-NMR (400 MHz, DMSO-d₆): δ = 7.95 (d, 2H), 7.73 (t, 1H), 7.53 (d, 2H), 7.51 (m, 1H), 7.42 (d, 2H), 7.30 (t, 2H), 7.25 (m, 1H), 6.85 (d, 1H), 4.38 (s, 2H).

### Beispiel 5A

### 4-[5-(6-Aminopyridin-2-yl)-1,2,4-oxadiazol-3-yl]benzosulfonylchlorid

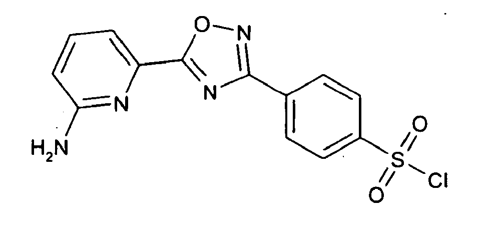

6-{3-[4-(Benzylthio)phenyl]-1,2,4-oxadiazol-5-yl}pyridin-2-amin (35.95 g) wird in einem Gemisch aus Essigsäure (200 ml) und Wasser (100 ml) in einem Eisbad auf 5°C abgekühlt. Chlor wird nach und nach eingeleitet bis das Ausgangsmaterial komplett umgesetzt ist (HPLC-Kontrolle), wobei die Temperatur 10°C nicht überschreiten darf. Das Reaktionsgemisch wird 15 min bei 5°C nachgerührt und dann mit Eiswasser (200 ml) verdünnt. Das Rohprodukt wird abfiltriert, mit Eiswasser (dreimal) und Diethylether (dreimal) gewaschen und anschließend im Vakuum getrocknet. Man erhält 26.00 g (85% d. Th.) des Produktes als Feststoff.
LC-MS (Methode 3): Rₜ = 2.22 min
MS (ESI): m/z = 337 [M+H]⁺

### Beispiel 6A

### 2-Chlor-5-fluor-1,3-dinitrobenzol

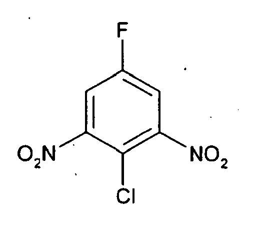

Zu 4-Fluor-2-6-dinitrophenol (26.00 g) in Benzol (50 ml) werden nacheinander DMF (10 ml) und Thionylchlorid (14 ml) zugegeben. Die resultierende Lösung wird 5 min bei RT gerührt (ein Zwischenprodukt fällt aus) und dann 1.5 Stunden unter Rückfluss (oder bis sich das Ausgangsmaterial komplett umgesetzt hat) erhitzt. Der Reaktionsgemisch wird auf RT abgekühlt, eingeengt und der Rückstand wird auf Eis/Wasser gegossen. Der Niederschlag wird abfiltriert, dreimal mit Wasser gewaschen und getrocknet. Nach der Umkristallisation aus Ethanol erhält man 23.50 g (83% d. Th.) des Produktes in kristalliner Form.
¹H-NMR (400 MHz, DMSO-d₆): δ = 8.56 (d, 2H).

### Beispiel 7A

### 5-Fluor-1,3-amino-benzol

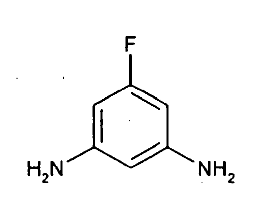

Zu 2-Chlor-5-fluor-1,3-dinitrobenzol (10.00 g) in Methanol (450 ml) werden Triethylamin (12.6 ml) und Palladium (10%ig auf Kohle) (6.0 g) zugegeben. Das Reaktionsgemisch wird unter 3 bar Wasserstoffdruck bei RT hydriert bis das Ausgangsmaterial komplett umgesetzt ist (2 Stunden). Der Ansatz wird über Celite filtriert und eingeengt. Der Rückstand wird in DCM (150 ml) aufgenommen und mit 10%iger Zitronensäurelösung behandelt. Die wässrige Phase wird anschließend mit 2N Natronlauge basisch gestellt und mit DCM (dreimal je 100 ml) extrahiert. Die organische Phase wird über Natriumsulfat getrocknet und eingeengt. Man erhält 5.0 g (88% d. Th.) des Produktes als ein Öl.
LC-MS (Methode 4): Rₜ =0.58 min
MS (ESI): m/z = 127 [M+H]⁺

### Beispiel 8A

### N-(3-Amino-5-fluorphenyl)-1-cyanocyclopropancarboxamid

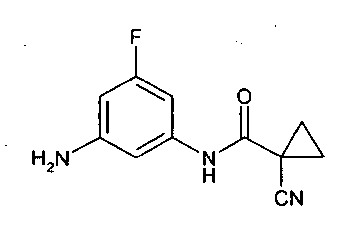

Zu 1-Cyanocyclopropancarbonsäure (2.05 g) in THF wird 1,1-Carbonyldiimidazol (3.29 g) zugegeben und die resultierende Lösung 45 min bei RT nachgerührt. Es wird 5-Fluor-1,3-amino-benzol (3.00 g) zugesetzt und weitere 2.5 Stunden gerührt. Anschließend wird die Reaktionslösung eingeengt, der Rückstand in DCM (150 ml) aufgenommen und mit Wasser gewaschen. Die wässrige Phase wird zweimal mit DCM extrahiert. Die organischen Extrakte werden zusammengegeben, über Natriumsulfat getrocknet und eingeengt. Der Rückstand wird über Kieselgel chromatographiert (Eluent DCM bis DCM-Methanol 50:1). Nach dem Einengen der relevanten Fraktion werden 2.35 g (58% d. Th.) Produkt isoliert.
LC-MS (Methode 1): Rₜ = 1.31 min
MS (ESI): m/z = 220 [M+H]⁺

### Beispiel 9A

### N-(3-{[(4-Cyanophenyl)sulfonyl]amino}phenyl)acetamid

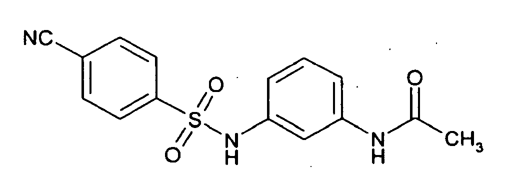

3'-Aminoacetanilid (13.54 g) wird in 2-Propanol (200 ml) gelöst und mit einer Lösung von Natriumacetat (8.51 g) in Wasser (100 ml) bei RT versetzt. 4-Cyanobenzosulfonylchlorid (20.0 g) wird zugegeben, das Reaktionsgemisch wird auf 30°C erwärmt und 3 Stunden bei RT nachgerührt. Der Ansatz wird auf Eis (250 ml) gegossen, der resultierende Feststoff abfiltriert, mit Wasser (dreimal) gewaschen und anschließend im Trockenschrank getrocknet. Man erhält 27.8 g (98% d. Th.) Produkt als Feststoff.
LC-MS (Methode 1): Rₜ = 1.79 min
MS (ESI): m/z = 316 [M+H]⁺
¹H-NMR (400 MHz, DMSO-d₆): δ = 10.52 (s, 1H), 9.94 (s, 1H), 8.05 (d, 2H), 7.90 (d, 2H), 7.46 (s, 1H), 7.27 (d, 1H), 7.13 (t, 1H), 6.72 (d, 1H), 2.00 (s, 3H).

### Beispiel 10A

### N-{3-[({4-[(Z)-Amino(hydroxyimino)methyl]phenyl}sulfonyl)amino]phenyl}acetamid

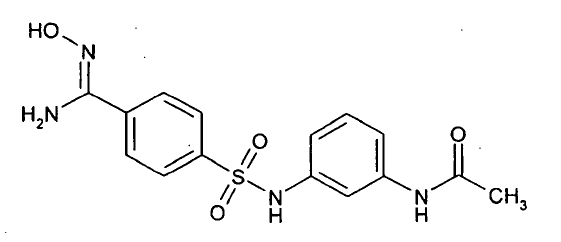

N-(3-{[(4-Cyanophenyl)sulfonyl]amino}phenyl)acetamid (27.00 g) wird in Ethanol (190 ml) vorgelegt, und nacheinander werden Hydroxylamin Hydrochlorid (7.14 g) und Triethylamin (14.0 ml) dazugegeben. Das Reaktionsgemisch wird 2 Stunden bei 50°C nachgerührt und anschließend auf Eis gegossen, abfiltriert und im Vakuumschrank getrocknet. Man erhält 25.78 g (86% d. Th.) Produkt als Feststoff.
LC-MS (Methode 1): Rₜ= 1.14 min
MS (ESI): m/z = 349 [M+H]⁺

### Beispiel 11A

### N-(6-{3-[4-({[3-(Acetylamino)phenyl]amino}sulfonyl)phenyl]-1,2,4-oxadiazol-5-yl} pyridin-2-yl)acetamid

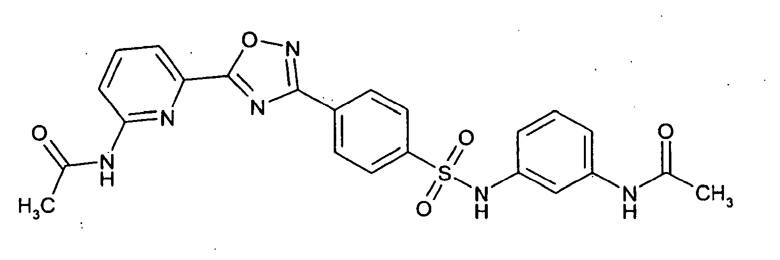

Zu 6-Acetylaminopyridin-2-carbonsäure (10.86 g) in einem Gemisch aus Dioxan (100 ml) und DMF (60 ml) wird 1,1-Carbonyldiimidazol (9.78 g) gelöst in Dioxan (100 ml) zugetropft und 3 Stunden bei RT nachgerührt. Danach wird N-{3-[({4-[(Z)-Amino(hydroxyimino)methyl]phenyl}sulfonyl)amino]phenyl}acetamid als Feststoff zugegeben und das Reaktionsgemisch 16 Stunden bei RT nachgerührt. Anschließend wird das Reaktionsgemisch 4 Stunden bei 100°C gerührt und danach auf Eis/Wasser gegossen. Das Produkt wird 10 Minuten stehengelassen, abfiltriert, mit Wasser (dreimal) gewaschen und im Vakuumofen getrocknet. Man erhält 25.55 g (90% d. Th.) Produkt als Feststoff.
HPLC (Methode 5): Rₜ = 4.03 min
MS (ESI): m/z = 493 [M+H]⁺

### Beispiel 12A

### N-(3-Aminophenyl)-4-[5-(6-aminopyridin-2-yl)-1,2,4-oxadiazol-3-yl]benzosulfonamid

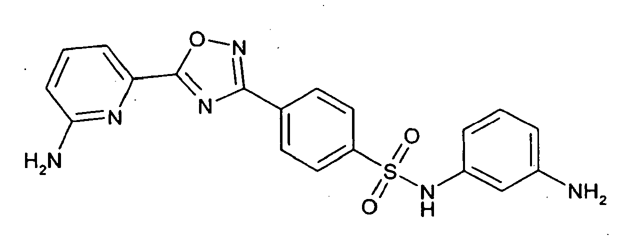

Zu N-(6-{3-[4-({[3-(Acetylamino)phenyl]amino}sulfonyl)phenyl]-1,2,4-oxadiazol-5-yl}pyridin-2-yl)acetamid (20.00 g) in Ethanol (200 ml) wird 15%ige Salzsäure (150 ml) zugesetzt. Das Reaktionsgemisch wird 6 Stunden unter Rückfluss gerührt, und anschließend in der Wärme mit 10%iger Natronlauge auf pH = 4 eingestellt. Man kühlt auf 5°C ab und rührt 16 Stunden nach. Das Rohprodukt wird abgesaugt, mit Wasser (zweimal) gewaschen und anschließend getrocknet. Man erhält 12.73 g (77 % d. Th.) Produkt als Feststoff.
HPLC (Methode 5): Rₜ = 3.53 min
MS (ESI): m/z = 409 [M+H]⁺
¹H-NMR (300 MHz, DMSO-d₆): δ = 10.16 (br s, 1H), 8.23 (d, 2H), 7.97 (d, 2H), 7.63 (t, 1H), 7.45 (d, 1H), 6.85 (t, 1H), 6.74 (d, 1H), 6.58 (br s, 2H), 6.42 (s, 1H), 6.28 (t, 1H), 5.44 (br s, 2H).

### Beispiel 13A

### N-(6-Brompyridin-2-yl)acetamid

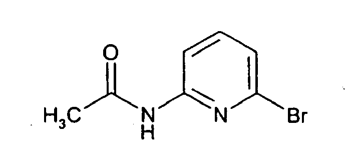

2-Amino-6-brompyridin (5.40 g) und Acetylchlorid (2.66 ml) werden in Methylenchlorid (80 ml) vorgelegt und auf 0°C gekühlt. Dann wird tropfenweise Triethylamin (6.53 ml) zugefügt und im Anschluss unter Rühren auf Raumtemperatur erwärmt. Der Ansatz wird mit 10%iger Natriumhydrogencarbonat-Lösung versetzt und mit Methylenchlorid extrahiert. Die organische Phase wird mit Wasser und gesättigter Natriumchlorid-Lösung gewaschen, über Natriumsulfat getrocknet und eingeengt. Nach Flashchromatographie (Eluent Methylenchlorid/Methanol 1:0, 500:1) erhält man 5.84 g (86% d. Th.) Produkt.
HPLC (Methode 6): Rₜ = 3.66 min

MS (DCI/NH₃): m/z = 215 und 217 [M+H]⁺, 232 und 234 [M+NH4]⁺, 249 und 251 (M+NH₄+NH₃)⁺
¹H-NMR (400 MHz, DMSO-d₆): δ = 10.79 (s, 1H, NH), 8.08 (d, 1H), 7.71 (t, 1H), 7.31 (d, 1H), 2.09 (s, 3H).

### Beispiel 14A

### N-[6-(3-Hydroxy-3-methylbut-1-in-1-yl)pyridin-2-yl]acetamid

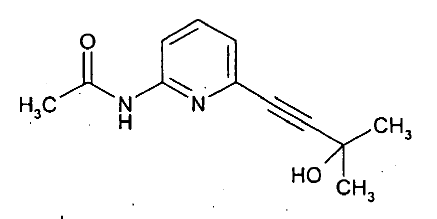

N-(6-Brompyridin-2-yl)acetamid (5.84 g) wird in Diethylamin (50 ml) vorgelegt. Nach dem Zufügen von 2-Methyl-3-butin-2-ol (2.51 g), Bis(triphenylphosphin)palladium(II)chlorid (381 mg) und Kupfer(I)iodid (52 mg) wird 2h bei Raumtemperatur gerührt. Dann wird der Ansatz eingeengt und flashchromatographiert (Eluent Methylenchlorid/Methanol 200:1, 100:1, 50:1). Man erhält 5.20 g (88% d. Th.) Produkt.
HPLC (Methode 6): Rₜ = 3.30 min
MS (Methode M-40, DCl/NH₃): m/z = 219 [M+H]⁺
¹H-NMR (400 MHz, DMSO-d₆): δ = 10.68 (s, 1H, NH), 8.05 (d, 1H), 7.75 (t, 1H), 7.14 (d, 1H), 5.55 (s, 1H, OH), 2.07 (s, 3H), 1.46 (s, 6H).

### Beispiel 15A

### N-(6-Ethinylpyridin-2-yl)acetamid

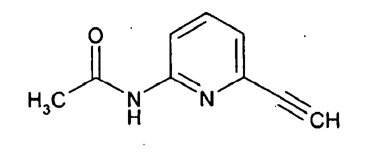

In Toluol (50 ml) wird N-[6-(3-Hydroxy-3-methylbut-1-in-1-yl)pyridin-2-yl]acetamid (5.20 g) vorgelegt, mit Natriumhydrid (95 mg) versetzt und 1.5h bei 120°C gerührt. Der Ansatz wird eingeengt, der Rückstand mit Wasser verdünnt und mit Essigsäureethylester extrahiert. Die organische Phase wird über Natriumsulfat getrocknet, eingeengt und flashchromatographiert (Eluent Methylenchlorid/Methanol 1:0, 500:1, 200:1, 100:1). Man erhält 1.75 g (43% d. Th.) Produkt.
HPLC (Methode 6):Rₜ = 3.18 min
MS (Methode M-40, DCI/NH₃): m/z = 161 [M+H]⁺, 178 [M+NH₄]⁺,
¹H-NMR (400 MHz, DMSO-d₆): δ = 10.68 (s, 1H, NH), 8.10 (d, 1H), 7.78 (t, 1H), 7.26 (d, 1H), 4.31 (s, 1H), 2.08 (s, 3H).

### Beispiel 16A

### N-(3-{[(4-Iodphenyl)sulfonyl]amino}phenyl)acetamid

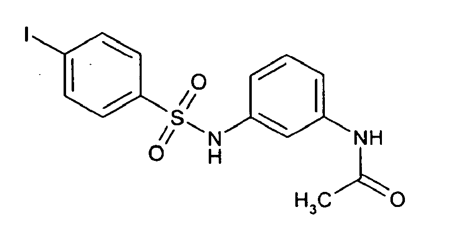

4-Iodbenzylsulfonsäurechlorid (10.0 g) wird in iso-Propanol (100 ml) vorgelegt, mit in wenig Wasser gelöstem Natriumacetat (3.12 g) versetzt und 30 min. bei Raumtemperatur gerührt. Dann wird N-(3-Aminophenyl)acetamid (4.96 g) zugefügt und weiterhin über Nacht gerührt. Der Ansatz wird mit Wasser und gesättigter Natriumchlorid-Lösung verdünnt und mit Essigsäureethylester extrahiert. Die organische Phase wird über Natriumsulfat getrocknet, eingeengt und flashchromatographiert (Eluent Methylenchlorid/Methanol 1:0, 100:1, 80:1). Man erhält 9.62 g (70% d. Th.) Produkt.
HPLC (Methode 6): Rₜ = 4.14 min
MS (ES⁺, ES⁻): m/z = 417 [M+H]⁺, 415 [M-H],
¹H-NMR (400 MHz, DMSO-d₆): δ = 10.31 (s, 1H, NH), 9.91 (s, 1H, NH), 7.93 (d, 2H), 7.51 (d, 2H), 7.45 (s, 1H), 7.26 (d, 1H), 7.12 (t, 1H), 6.73 (d, 1H), 2.00 (s, 3H).

### Beispiel 17A

### N-(6-{[4-({[3-(Acetylamino)phenyl]amino}sulfonyl)phenyl]ethinyl}pyridin-2-yl)acetamid

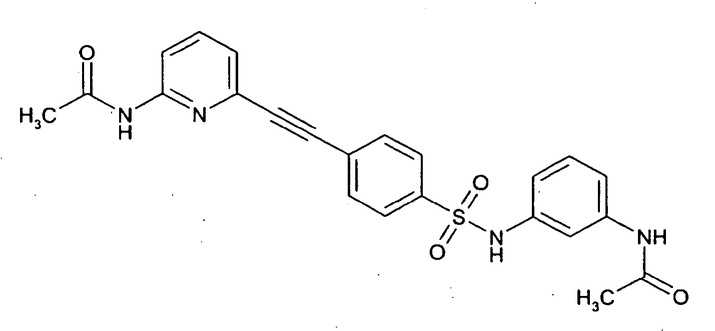

In einer Argonatmosphäre werden N-(3-{[(4-Iodphenyl)sulfonyl]amino}phenyl)acetamid (4.60 g), Tetrakis(triphenylphosphin)palladium(0) (1.28 g) und Kupfer(I)iodid (421 mg) in DMF vorgelegt, mit N-(6-Ethinylpyridin-2-yl)acetamid (2.66 g) und Triethylamin (15.4 ml) versetzt und 2h bei Raumtemperatur gerührt. Dann wird mit Wasser verdünnt, in Methylenchlorid extrahiert, die organische Phase getrocknet und flashchromatographiert (Eluent Methylenchlorid/Methanol 1:0, 200:1, 100:1, 50:1, 30:1). Es werden 3.56 g (48% d. Th.) Produkt erhalten.
HPLC (Methode 6): Rₜ = 3.86 min
MS (ES⁺, ES⁻): m/z = 449 [M+H]⁺, 447 [M-H]⁻,
¹H-NMR (400 MHz, DMSO-d₆): δ = 10.76 (s, 1H, NH), 10.38 (s, 1H, NH), 9.93 (s,1H, NH), 8.13 (d, 1H), 7.88-7.78 (m, 3H), 7.73 (d, 2H), 7.48 (s, 1H), 7.37 (d, 1H), 7.26 (d, 1H), 7.12 (t,1H), 6.76 (d, 1H), 2.09 (s, 3H), 2.00 (s, 3H).

### Beispiel 18A

### N-(3-Aminophenyl)-4-[(6-aminopyridin-2-yl)ethinyl]benzosulfonamid Dihydrochlorid

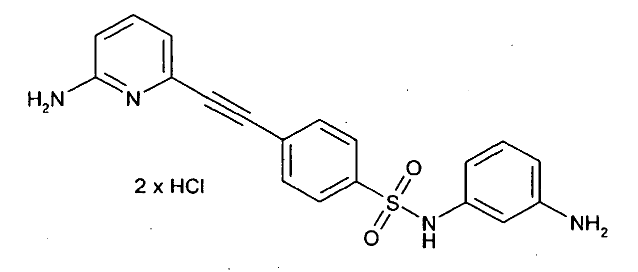

N-(6-{[4-({[3-(Acetylamino)phenyl]amino}sulfonyl)phenyl]ethinyl}pyridin-2-yl)acetamid (3.12 g) wird in Ethanol (45 ml) vorgelegt, mit 20%iger Salzsäure (45 ml) versetzt und 3h bei 60°C gerührt. Der Ansatz wird eingeengt und der Rückstand mit Acetonitril ausgerührt. Nach dem Absaugen, weiterem Waschen mit Acetonitril und Trocknen am Hochvakuum erhält man 3.45 g (quant.) Produkt.
HPLC (Methode 6):Rₜ = 3.65 min
MS (ES⁺, ES⁻): m/z = 365 [M+H]⁺, 363 [M-H]⁻,
1H-NMR (400 MHz, DMSO-d6): δ = 10.70 (s, 1H, NH), 7.91-7.78 (m, 5H), 7.24 (t, 1H), 7.09 (d, 1H), 7.02 (s, 1H), 6.96-6.83 (m, 3H).

### Beispiel 19A

### 4-[5-(6-Acetylaminopyridin-2-yl)-1,2,4-oxadiazol-3-yl]benzosulfonylchlorid

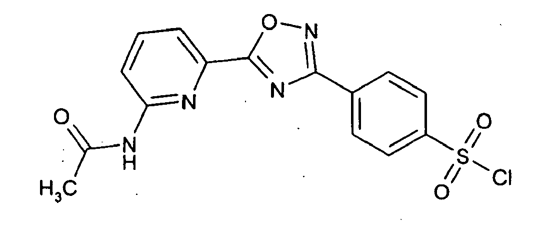

N-(6-{3-[4-(Benzylthio)phenyl]-1,2,4-oxadiazol-5-yl}pyridin-2-yl)acetamid (11.55 g) wird in einem Gemisch aus Essigsäure (80 ml) und Wasser (50 ml) in einem Eisbad gerührt und auf 5°C abgekühlt. Chlor wird nach und nach eingeleitet bis das Ausgangsmaterial komplett umgesetzt ist (HPLC-Kontrolle), wobei die Temperatur 10°C nicht überschreiten darf. Das Reaktionsgemisch wird 15 min bei 5°C nachgerührt und dann mit Eiswasser (100 ml) verdünnt. Das Rohprodukt wird abfiltriert, mit Eiswasser (dreimal) und Diethylether (dreimal) gewaschen und anschließend im Vakuum getrocknet. Man erhält 9.60 g (88% d. Th.) des Produktes als Feststoff.
LC-MS (Methode 3): Rₜ = 2.31 min
MS (ESI): m/z = 379 [M+H]⁺

### Ausführungsbeispiele

### Beispiel 1

### N-(3-[({4-[5-(6-Aminopyridin-2-yl)-1,2,4-oxadiazol-3-yl]phenyl}sulfonyl)amino]-5-fluorphenyl}-1-cyanocyclopropancarboxamid

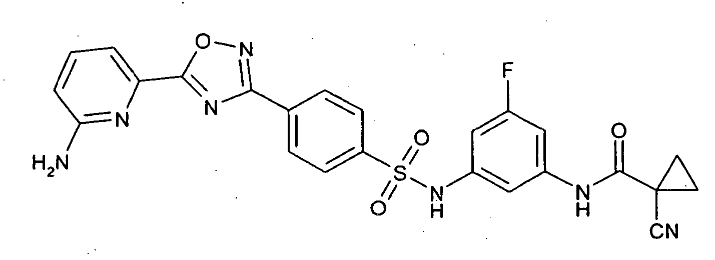

Zu 4-[5-(6-Aminopyridin-2-yl)-1,2,4-oxadiazol-3-yl]benzosulfonylchlorid (3.78 g) in trockenem Pyridin (120 ml) wird N-(3-Amino-5-fluorphenyl)-1-cyanocyclopropancarboxamid (2.46 g) zugegeben. Die resultierende Lösung wird 18 Stunden bei RT gerührt, und anschließend auf Eis/Wasser gegossen. Das Rohprodukt wird abfiltriert, mit Wasser gewaschen und getrocknet. Nach Chromatographie über Kieselgel (Methylenchlorid bis Methylenchlorid/Methanol 50:1) und einengen der relevanten Fraktionen werden 2.28 g (40% d. Th.) Produkt isoliert.
LC-MS (Methode 3): Rₜ = 2.07 min
MS (ESI): m/z = 520 [M+H]⁺
¹H-NMR (400 MHz, DMSO-d₆): δ = 8.37 (d, 2H), 8.01 (d, 2H), 7.64 (t, 1H), 7.45 (d, 1H), 7.35 (s, 1H), 7.21 (br d, 1H), 6.73 (d, 1H), 6.68 (br d, 1H), 6.56 (br s, 2H), 1.65 (s, 4H).

### Beispiel 2

### N-{3-[({4-[5-(6-Aminopyridin-2-yl)-1,2,4-oxadiazol-3-yl]phenyl}sulfonyl)amino]phenyl}-1-cyanocyclopropancarboxamid

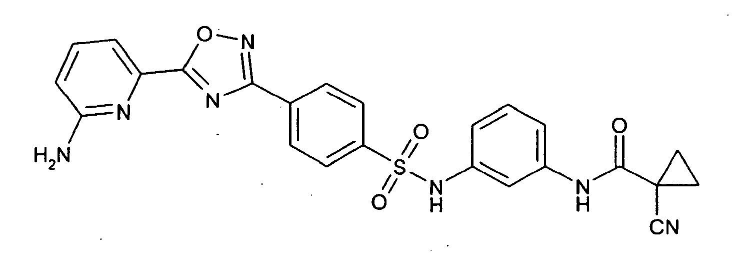

N-(3-Aminophenyl)-4-[5-(6-aminopyridin-2-yl)-1,2,4-oxadiazol-3-yl]benzosulfonamid (4.50 g) wird in trockenem DMF (110 ml) vorgelegt, HATU (6.28 g), 1-Cyanocyclopropancarbonsäure (2.45 g) und N,N-Diisopropylethylamin (2.90 ml) werden zugegeben, und das Reaktionsgemisch wird 1h unter Argon bei RT gerührt und anschließend eingeengt. Der Rückstand wird über Kieselgel chromatographiert (Eluent Methylenchlorid/Methanol 100:1 bis 20:1). Nach Einengen der relevanten Fraktionen können 4.99 g (90% d. Th.) Produkt isoliert werden.
LC-MS (Methode 2): Rₜ = 2.13 min
MS (ESI): m/z = 502 [M+H]⁺
¹H-NMR (400 MHz, DMSO-d₆): δ = 10.47 (s, 1H), 10.06 (s, 1H), 8.23 (d, 2H), 7.97 (d, 2H), 7.64 (t, 1H), 7.53 (s, 1H), 7.45 (d, 1H), 7.28 (d, 1H), 7.17 (t, 1H), 6.84 (d, 1H), 6.73 (d, 1H), 1.65 (s, 4H).

### Beispiel 3

### N-{3-[({4-[5-(6-Aminopyridin-2-yl)-1,2,4-oxadiazol-3-yl]phenyl}sulfonyl)amino]-2-methylphenyl}-1-cyanocyclopropancarboxamid

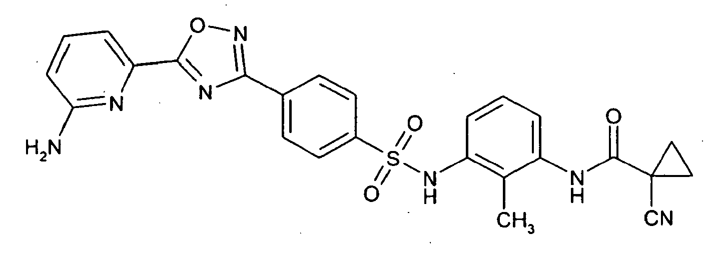

Die Herstellung erfolgt analog zu Beispiel 2 ausgehend von 3'-Amino-2'-methylphenylacetamid.
LC-MS (Methode 3): Rₜ = 1.91 min
MS (ESI): m/z = 516 [M+H]⁺
¹H-NMR (400 MHz, DMSO-d₆): δ = 9.91 (s, 1H), 9.67 (s, 1H), 8.26 (d, 2H), 7.87 (d, 2H), 7.65 (t,1H), 7.47 (d, 1H), 7.10 (m, 2H), 6.84 (dd, 1H), 6.76 (d, 1H), 1.91 (s, 3H), 1.63 (m, 4H).

### Beispiel 4

### N-{3-[({4-[(6-Aminopyridin-2-yl)ethinyl]phenyl)sulfonyl)amino]phenyl}-1-cyanocyclopropancarboxamid Hydrochlorid

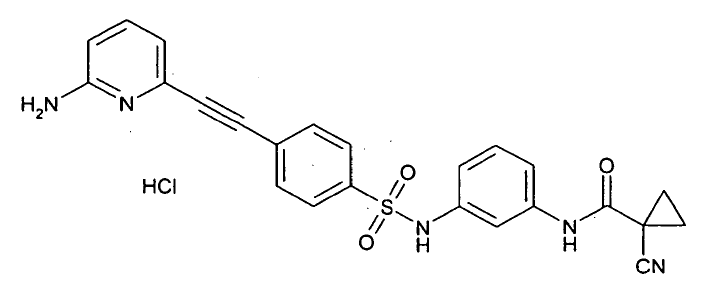

N-(3-Aminophenyl)-4-[(6-aminopyridin-2-yl)ethinyl]benzosulfonamid Dihydrochlorid (750 mg), 1-Cyanocyclopropansäure (229 mg), HATU (783 mg) und N,N-Diisopropylethylamin (1.04 ml) werden in trockenem DMF (7 ml) über Nacht bei Raumtemperatur gerührt. Der Ansatz wird direkt per präparativer HPLC (Eluent Wasser (mit 1% Salzsäure)/Acetonitril, Fluss 50 ml/min.) aufgereinigt, und man erhält 400 mg (47% d. Th.) Produkt.
HPLC (Methode 6): Rₜ = 3.87 min
MS (ES⁺, ES⁻): m/z = 458 [M-HCl+H]⁺, 456 [M-HCl-H]⁻,
¹H-NMR (400 MHz, DMSO-d₆): δ = 10.45 (s, 1H, NH), 10.07 (s, 1H, NH), 7.83 (d, 2H), 7.79-7.66 (m, 3H), 7.51 (s, 1H), 7.27 (d, 1H), 7.17 (t, 1H), 7.01 (d, 1H), 6.82 (d, 2H), 1.65 (s, 4H).

### Beispiel 5

### N-{3-[({4-[5-(6-Acetylaminopyridin-2-yl)-1,2,4-oxadiazol-3-yl]phenyl}sulfonyl)amino]-5-fluorphenyl}-1-cyanocyclopropancarboxamid

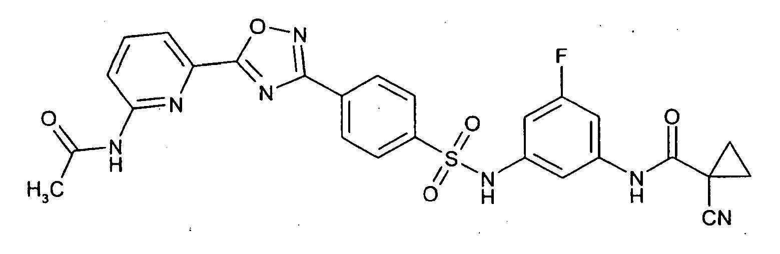

Zu 4-[5-(6-Acetylaminopyridin-2-yl)-1,2,4-oxadiazol-3-yl]benzosulfonylchlorid (148 mg) in trockenem Pyridin (2 ml) wird N-(3-Amino-5-fluorphenyl)-1-cyanocyclopropancarboxamid (100 mg) zugegeben. Die resultierende Lösung wird 18 Stunden bei RT gerührt, und anschließend auf Eis/Wasser gegossen. Das Rohprodukt wird abfiltriert, mit Wasser gewaschen und getrocknet. Nach präparativer RP-HPLC (Eluent Acetonitril:Wasser Gradient) und einengen der relevanten Fraktionen werden 53 mg (24% d. Th.) Produkt isoliert.
LC-MS (Methode 7): Rₜ = 2.46 min
MS (ESI): m/z = 562 [M+H]⁺
1H-NMR (400 MHz, DMSO-d6): δ = 10.99 (s, 1H), 10.78 (s, 1H),10.26 (s, 1H), 8.40 (d, 1H), 8.17 (d, 2H), 8.06 (m, 4H), 7.37 (s, 1H), 7.21 (d, 1H), 6.68 (d, 1H), 2.15 (s, 3H), 1.66 (s, 4H).

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalo-Virus) Zytopathogenitätstests

Die Testverbindungen werden als 50 millimolare (mM) Lösungen in Dimethylsulfoxid (DMSO) eingesetzt. Ganciclovir^{®} dient als Referenzverbindung. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium, In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.003), d.h. 1-3 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5% CO₂ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50% im Vergleich zur unbehandelten Viruskontrolle hemmt;
SI (Selektivitätsindex) = CCso (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-Nr.** | **NHDF** **CC50 [µM]** | **HCMV** **EC50 [µM]** | **SI** **HCMV** |
|---|---|---|---|
| 1 | 71 | 0.011 | 6450 |
| 2 | 141 | 0.007 | 20140 |
| 3 | 102 | 0.002 | 51000 |
| 4 | 47 | 0.021 | 2240 |
| 5 | 71 | 0.026 | 2730 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

5-6 Wochen alte immundefiziente Mäuse (16 - 20 g), Fox Chase SCID.NOD oder NOD.CB17-Prkdc/J werden von kommerziellen Züchtern (Taconic M&B, Dänemark; Jackson, USA) bezogen. Die Tiere werden unter sterilen Bedingungen (einschließlich Streu und Futter) in Isolatoren gehalten.

### Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential Medium (MEM), 20% foetalem Kälberserum (FKS) (v/v), 1% Glutamin (v/v), 1% Pen/Strep (v/v) mit 10% DMSO bei -80°C aufbewahrt. Nach serieller Verdünnung der virusinfizierten Zellen in Zehnerschritten erfolgt die Titerbestimmung auf 24-Well-Platten konfluenter NHDF-Zellen nach Fixierung und Färbung mit einer Giemsa-Formaldehyd-Lösung.

### Vorbereitung der Schwämme. Transplantation. Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM, 10% FKS (v/v), 1% Glutamin (v/v), 1% Pen/Strep (v/v) aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10% FKS (v/v), 1% Glutamin (v/v), 1% Pen/Strep (v/v) auf einen feuchten Schwamm getropft. Die Schwämme werden 3-4 Stunden inkubiert, um das adherieren der Zellen zu ermöglichen. Anschließend werden die Schwämme nach Zugabe von Medium (MEM, 10% FKS) (v/v), 1% Glutamin (v/v), 1% Pen/Strep (v/v)) über Nacht inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber oder Klammern verschlossen. 4 - 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (9 Uhr) peroral mit Substanz behandelt. Die Tagesdosis beträgt beispielsweise 1 oder 3 oder 10 oder 30 oder 100 mg/kg Körpergewicht, das Applikationsvolumen ist 10 ml/kg Körpergewicht. Die Formulierung der Substanzen erfolgt in Form einer 0.5%-igen Tylosesuspension / PBS mit 2% DMSO oder einer anderen geeigneten Mischung, die die Löslichkeit der Substanzen unterstützt, z.B. 2 % Ethanol, 2.5% Solutol, 95.5% PBS. 10 Tage nach Transplantation und ca. 16 Stunden nach der letzten Substanzapplikation werden die Tiere schmerzlos getötet und der Schwamm entnommen. Die virusinfizierten Zellen werden durch Kollagenaseverdau (330 U/ 1.5 ml) aus dem Schwamm freigesetzt und in Gegenwart von MEM, 10% FKS (v/v), 1% Glutamin (v/v), 1% Pen/Strep (v/v), 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Fixierung und Färbung mit einer Giemsa-Formaldehyd-Lösung. Ermittelt wird die Anzahl infizierter Zellen bzw. infektiöser Viruspartikel (infectious center assay) nach Substanzbehandlung im Vergleich zur placebobehandelten Kontrollgruppe. Die statistische Auswertung erfolgt mittels geeigneter Computerprogramme, z.B. GraphPad Prism.

### Pharmakokinetische Untersuchungen.

Die Pharmakokinetik der Wirksubstanzen wird nach intravenöser und oraler Gabe von Dosen im Bereich von 1 mg/kg i.v. und 3 mg/kg p.o. an jeweils drei männlichen Wistar-Ratten pro Verabreichungsroute untersucht. Um eine wiederholte Blutentnahme zu ermöglichen, wird den Tieren am Tag vor dem Experiment ein Katheter in die Jugularvene implantiert. Die Substanzen werden sowohl intravenös als auch peroral als Lösung verabreicht. Dabei wird zumeist für die intravenöse Gabe eine Plasmaformulierung verwendet (Rattenplasma mit 1-2% Ethanol oder DMSO, 2 ml/kg) und für die perorale Gabe eine PEG Formulierung (10% Ethanol, 40% PEG 400, 50% Wasser, 5 ml/kg).

Nach der Verabreichung der Wirksubstanz werden über 24 h Blutproben über den Katheter in mit Heparin versehenen Probenröhrchen gesammelt. Im Anschluss an die Blutentnahme werden die Blutproben zentrifugiert und der Plasmaüberstand in Eppendorfröhrchen pipettiert. Die Plasmaproben werden bei mindestens -15°C aufbewahrt, bis die Analyse vorgenommen wird.

Zur Aufarbeitung werden die Proben aufgetaut. Anschließend werden die Plasmaproteine durch Zugabe von Acetonitril, das mit einem internen Standard versehen ist, gefällt. Als interner Standard wird eine strukturell dem Wirkstoff möglichst ähnliche Substanz aus der gleichen Strukturklasse gewählt. Zur Herstellung von Kalibrierproben werden Aliquots an Leerplasma mit unterschiedlichen Konzentrationen der Wirksubstanz versetzt und gemeinsam mit den unbekannten Proben aufgearbeitet. Zusätzlich werden Qualitätskontrollproben mit drei verschiedenen Konzentrationen hergestellt, die der Validierung der Analytik dienen.

Die Bestimmung der Wirksubstanz in den Proben erfolgt durch Hochleistungsflüssigkeits-Chromatographie mit massenspektrometrischer Detektion (LC/MS-MS). Die Wirksubstanzkonzentrationen in den unbekannten Proben werden auf Basis ihrer relativen Peak-Höhen oder -Flächen im Vergleich zur Kalibrierkurve mit dem Programm Concalc for Windows (CCW, Integrierte Labordatensysteme, Version 2.5 oder nachfolgende, Bayer AG) bestimmt. Anschließend werden aus den tierindividuellen Plasmakonzentrations-Zeitverläufen die pharmakokinetischen Parameter durch nicht-kompartimentelle Analyse mit Hilfe des Programms KINCALC, Version 2.50.02 (Bayer AG, 2001), errechnet.

Die Verbindungen, die das gewünschte, verbesserte pharmakokinetische Profil an der Ratte zeigen, werden im Anschluss auch nach Gabe an Mäuse und Hunde pharmakokinetisch untersucht. Auf Basis aller dieser Daten wird eine erste Abschätzung der humanen Pharmakokinetik durch ein Inter-Spezies Upscaling nach Boxenbaum durchgeführt.

Folgende Daten lassen sich aus diesen Tests ermitteln:
- Vₛₛ:: Verteilungsvolumen
- CL:: Eliminationsgeschwindigkeit
- t_{1/2}:: Halbwertszeit
- AUC:: Gesamtfläche unter der Kurve der Wirkstoffkonzentration über die Zeit
- Cₘₐₓ:: maximale Konzentration
- F:: Bioverfügbarkeit

Pharmakokinetische Daten für die Verbindung vom Beispiel 1 nach einmaliger intravenöser und peroraler Gabe an männliche Wistar Ratten (n = 3 pro Zeitpunkt bzw. n=3) sind in der Tabelle B dargestellt. Die Verbindungen der Erfindung zeigen ein verbessertes pharmakokinetisches Verhalten.

**Tabelle B**

| **Wistar Ratte** | | |
|---|---|---|
| Dosis i.v. | | 1.3 mg/kg i.v.¹ |
| Vₛₛ | [l/kg] | 0.321 |
| CL_{Plasma} | [l/(h·kg)] | 0.064 |
| CL_{Blut} | [l/(h·kg)] | 0.128 |
| t_{½} | [h] | 4.26 |

| Dosis p.o. | | 3 mg/kg p.o.² |
|---|---|---|
| AUC_{norm,po.} | [kg·h/l] | 8.58 |
| C_{max,norm,p.o}. | [kg/l] | 1.08 |
| F | [%] | 55.3 |

| | | |
|---|---|---|
| ¹: Lösung in Rattenplasma mit 1% DMSO, 2 ml/kg ²: Lösung in 10% Ethanol, 40% PEG 400, 50% Wasser, 5 ml/kg | | |

### Identifikation von Metaboliten

Speziesunterschiede im Metabolismus eines Wirkstoffs können einen großen Einfluss auf dessen Entwickelbarkeit haben. Ziel ist es Substanzen zu finden die sich in den metabolischen Abbauwegen zwischen Mensch und gängigen Tox-Spezies wie z.B. Ratte und Hund nicht signifikant unterscheiden. Dazu werden neue Wirkstoffe in vitro zuerst mit Lebermikrosomen von Ratte, Hund und Mensch inkubiert zum Vergleich des Phase I Metabolismus. Anschließend werden weiterhin interessante Verbindungen noch zusätzlich in Hepatozyten von Ratte und Mensch inkubiert um einen kompletten hepatischen Phase I und Phase II Metabolismus zu erhalten und zu vergleichen.

Alle neuen Wirkstoffe werden mit einer Konzentration von 20 µM inkubiert. Dazu werden Stammlösungen mit einer Konzentration von 2 mM in Acetonitril hergestellt, welche dann mit einer 1:100 Verdünnung in den Inkubationsansatz pipettiert werden, um maximal 1% Acetonitril im Ansatz zu haben. Die Lebermikrosomen werden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten werden in Suspension in Williams E Medium ebenfalls bei 37°C inkubiert. Nach einer Inkubationszeit von 0 - 4h werden die Inkubationsansätze mit Acetonitril abgestoppt (Endkonzentration ca. 30%) und das Protein bei ca. 15000 x g abzentrifugiert. Die so abgestoppten Proben werden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS). Dazu werden die Überstände der Inkubationsproben mit geeigneten C18-reversed-phase Säulen und variablen Gemischen Acetonitril und 10 mM Ammoniumformiat chromatographiert. Die UV-Chromatogramme in Verbindung mit massenspektrometrischen Daten dienen zur Identifizierung der Metabolite. Die so erzeugten Metabolitenprofile der einzelnen untersuchten Spezies werden verglichen und dienen zur Feststellung von Speziesunterschieden.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

10-500 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 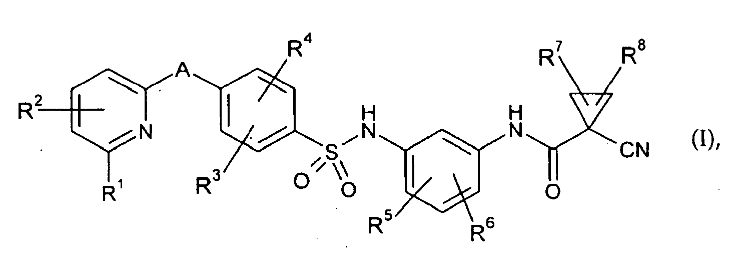 in welcher
A für eine Gruppe der Formel 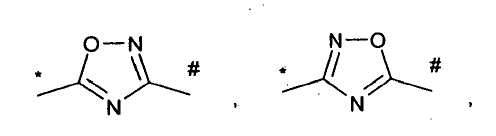 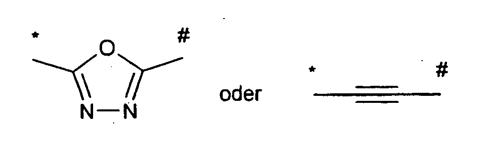 steht,
wobei
* die Anknüpfstelle an das Kohlenstoffatom des Pyridinyl-Rings ist,
und
# die Anknüpfstelle an das Kohlenstoffatom des Phenyl-Rings ist,
R¹ für Wasserstoff, Amino oder Methylcarbonylamino steht,
R² für Wasserstoff oder Halogen steht,
R³ für Wasserstoff, Halogen oder Cyano steht,
R⁴ für Wasserstoff, Halogen oder Cyano steht,
R⁵ für Wasserstoff oder Halogen steht,
R⁶ für Wasserstoff oder Halogen steht,
R⁷ für Wasserstoff, Halogen oder C₁-C₃-Alkyl steht,
R⁸ für Wasserstoff, Halogen oder C₁-C₃-Alkyl steht,
oder eines ihrer Salze, ihrer Solvate oder der Solvate ihrer Salze.

2. Verbindung nach Anspruch 1, **dadurch gekennzeichnet, dass**
A für eine Gruppe der Formel 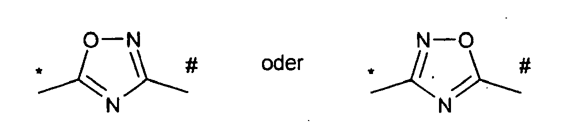 steht,
wobei
* die Anknüpfstelle an das Kohlenstoffatom des Pyridinyl-Rings ist,
und
# die Anknüpfstelle an das Kohlenstoffatom des Phenyl-Rings ist,
R¹ für Wasserstoff, Amino oder Methylcarbonylamino steht,
R², R³ und R⁴ für Wasserstoff stehen,
R⁵ für Wasserstoff oder Halogen steht,
R⁶ für Wasserstoff oder Halogen steht,
R⁷ und R⁸ für Wasserstoff stehen,
oder eines ihrer Salze, ihrer Solvate oder der Solvate ihrer Salze.

3. Verbindung nach Anspruch 1 oder 2, **dadurch gekennzeichnet, dass**
A für eine Gruppe der Formel 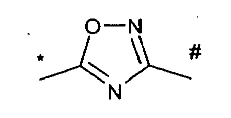 steht,
wobei
* die Anknüpfstelle an das Kohlenstoffatom des Pyridinyl-Rings ist,
und
# die Anknüpfstelle an das Kohlenstoffatom des Phenyl-Rings ist,
R¹ für Amino oder Methylcarbonylamino steht,
R², R³ und R⁴ für Wasserstoff stehen,
R⁵ für Wasserstoff steht,
R⁶ für Wasserstoff oder Halogen steht,
R⁷ und R⁸ für Wasserstoff stehen,
oder eines ihrer Salze, ihrer Solvate oder der Solvate ihrer Salze.

4. Verfahren zur Herstellung einer Verbindung der Formel (I) nach Anspruch 1, **dadurch gekennzeichnet, dass** eine Verbindung der Formel 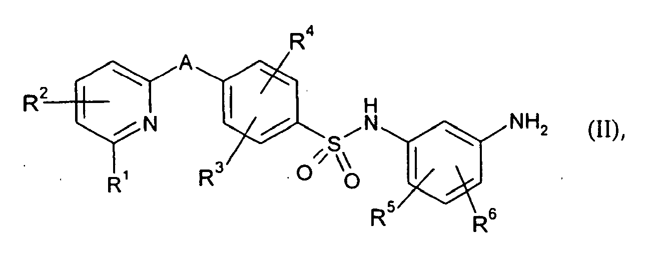 in welcher
A, R¹, R², R³, R⁴, R⁵ und R⁶ die in Anspruch 1 angegebene Bedeutung haben,
mit einer Verbindung der Formel 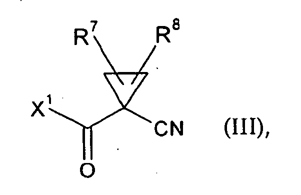 in welcher
R⁷ und R⁸ die in Anspruch 1 angegebene Bedeutung haben, und
X¹ für Halogen, bevorzugt Chlor oder Brom, oder Hydroxy steht,
umgesetzt wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit einem inerten, nichttoxischen, pharmazeutisch geeigneten Hilfsstoff

7. Verwendung einer Verbindung nach einem der Ansprüche 1 bis 3 zur Herstellung eines Arzneimittels zur Behandlung und/oder Prophylaxe von Virusinfektionen.

8. Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

9. Arzneimittel nach Anspruch 6 zur Behandlung und/oder Prophylaxe von Virusinfektionen.

10. Verwendung einer antiviral wirksamen Menge mindestens einer Verbindung nach einem der Ansprüche 1 bis 3, eines Arzneimittels nach Anspruch 6 oder eines nach Anspruch 7 oder 8 erhaltenen Arzneimittels zur Herstellung eines Medikaments zur Bekämpfung von Virusinfektionen in Menschen und Tieren.

## Claims

1. Compound of formula 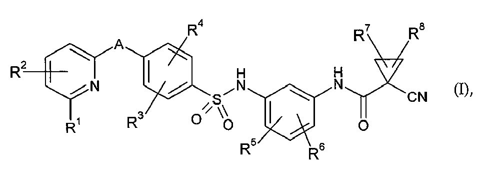 in which
A represents a group of formula 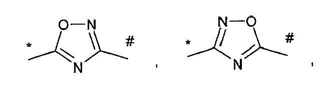 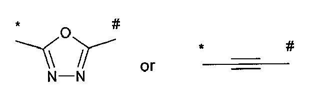 whereby
* is the linkage site to the carbon atom of the pyridinyl ring,
and
# is the linkage site to the carbon atom of the phenyl ring,
R¹ represents hydrogen, amino or methylcarbonylamino,
R² represents hydrogen or halogen,
R³ represents hydrogen, halogen or cyano,
R⁴ represents hydrogen, halogen or cyano,
R⁵ represents hydrogen or halogen,
R⁶ represents hydrogen or halogen,
R⁷ represents hydrogen, halogen or C₁-C₃-alkyl,
R⁸ represents hydrogen, halogen or C₁-C₃-alkyl,
or one of its salts, its solvates or the solvates of its salts.

2. Compound according to claim 1, **characterized in that**
A represents a group of formula 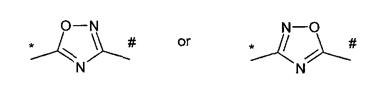 whereby
* is the linkage site to the carbon atom of the pyridinyl ring,
and
# is the linkage site to the carbon atom of the phenyl ring,
R¹ represents hydrogen, amino or methylcarbonylamino,
R², R³ and R⁴ represent hydrogen,
R⁵ represents hydrogen or halogen,
R⁶ represents hydrogen or halogen,
R⁷ and R⁸ represent hydrogen,
or one of its salts, its solvates or the solvates of its salts.

3. Compound according to claim 1 or 2, **characterized in that**
A represents a group of formula 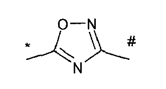 whereby
* is the linkage site to the carbon atom of the pyridinyl ring,
and
# is the linkage site to the carbon atom of the phenyl ring,
R¹ represents amino or methylcarbonylamino,
R², R³ and R⁴ represent hydrogen,
R⁵ represents hydrogen,
R⁶ represents hydrogen or halogen,
R⁷ and R⁸ represent hydrogen,
or one of its salts, its solvates or the solvates of its salts.

4. Method for the preparation of a compound of formula (I) according to claim 1, **characterized in that** a compound of formula 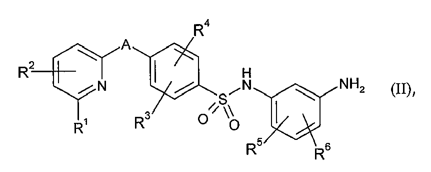 in which
A, R¹, R², R³, R⁴, R⁵ and R⁶ have the meaning indicated in claim 1,
is reacted with a compound of formula 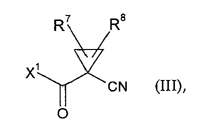 in which
R⁷ and R⁸ have the meaning indicated in claim 1, and
X¹ represents halogen, preferably chlorine or bromine, or hydroxy.

5. Compound according to any one of claims 1 to 3 for the treatment and/or prophylaxis of diseases.

6. Medicament comprising a compound according to any one of claims 1 to 3 in combination with an inert, non-toxic, pharmaceutically acceptable excipient.

7. Use of a compound according to any one of claims 1 to 3 for the production of a medicament for the treatment and/or prophylaxis of viral infections.

8. Use according to claim 7, **characterized in that** the viral infection is an infection with the human cytomegalovirus (HCMV) or another representative of the group of herpes viridae.

9. Medicament according to claim 6 for the treatment and/or prophylaxis of viral infections.

10. Use of an antivirally effective amount of at least one compound according to any one of claims 1 to 3, of a medicament according to claim 6 or of a medicament obtained according to claim 7 or 8 for the production of a medicament for controlling viral infections in humans and animals.

## Revendications

1. Composé de formule 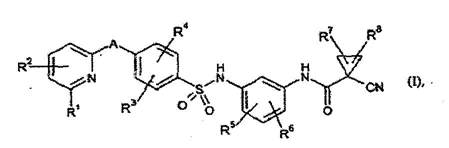 dans laquelle
A est un groupe de formule 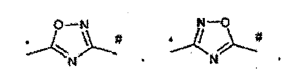 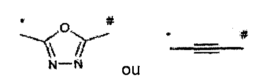 où
* est un site de liaison à l'atome de carbone du cycle pyridinyle,
et
# est un site de liaison à l'atome de carbone du cycle phényle,
R¹ est un hydrogène, un amino ou un méthylcarbonylamino,
R² est un hydrogène ou un halogène,
R³ est un hydrogène, un halogène ou un cyano,
R⁴ est un hydrogène, un halogène ou un cyano,
R⁵ est un hydrogène ou un halogène,
R⁶ est un hydrogène ou un halogène,
R⁷ est un hydrogène, un halogène ou un alkyle en C₁-C₃,
R⁸ est un hydrogène, un halogène ou un alkyle en C₁-C₃,
ou un de ses sels, solvates ou solvates de ses sels.

2. Composé selon la revendication 1, **caractérisé en ce que**
A est un groupe de formule 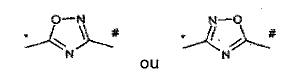 où
* est un site de liaison à l'atome de carbone du cycle pyridinyle,
et
# est un site de liaison à l'atome de carbone du cycle phényle,
R¹ est un hydrogène, un amino ou un méthylcarbonylamino,
R², R³ et R⁴ sont des hydrogènes,
R⁵ est un hydrogène ou un halogène,
R⁶ est un hydrogène ou un halogène,
R⁷ et R⁸ sont des hydrogènes,
ou un de ses sels, solvates ou solvates de ses sels.

3. Composé selon la revendication 1 ou 2, **caractérisé en ce que**
A est un groupe de formule 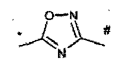 où
* est un site de liaison à l'atome de carbone du cycle pyridinyle,
et
# est un site de liaison à l'atome de carbone du cycle phényle,
R¹ est un amino ou un méthylcarbonylamino,
R², R³ et R⁴ sont des hydrogènes,
R⁵ est un hydrogène,
R⁶ est un hydrogène ou halogène,
R⁷ et R⁸ sont des hydrogènes,
ou un de ses sels, solvates ou solvates de ses sels.

4. Procédé de préparation d'un composé de formule (I) selon la revendication 1, **caractérisé en ce que** l'on fait réagir un composé de formule 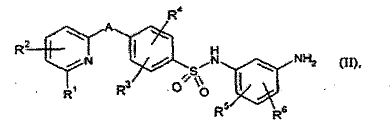 dans laquelle
A, R¹, R², R³, R⁴, R⁵ et R⁶ ont la signification indiquée dans la revendication 1,
avec un composé de formule 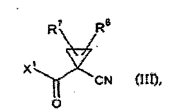 dans laquelle
R⁷ et R⁸ ont la signification indiquée dans la revendication 1, et
X¹ est un halogène, de préférence un chlore ou un brome, ou un hydroxy.

5. Composé selon l'une des revendications 1 à 3 pour le traitement et/ou la prophylaxie de maladies.

6. Médicament contenant un composé selon l'une des revendications 1 à 3 en combinaison avec un auxiliaire inerte non toxique, pharmaceutiquement acceptable.

7. Utilisation d'un composé selon l'une des revendications 1 à 3 pour la préparation d'un médicament destiné au traitement et/ou à la prophylaxie des infections virales.

8. Utilisation selon la revendication 7, **caractérisée en ce que** l'infection virale est une infection par le cytomégalovirus humain (HCMV) ou un autre représentant du groupe des *Herpes viridae.*

9. Médicament selon la revendication 6 pour le traitement et/ou la prophylaxie des infections virales.

10. Utilisation d'une quantité efficace sur le plan antiviral d'au moins un composé selon l'une des revendications 1 à 3, d'un médicament selon la revendication 6 ou d'un médicament obtenu selon la revendication 7 ou 8 pour la préparation d'un médicament destiné à lutter contre les infections virales chez les humains et les animaux.
